# EUROPEAN PATENT APPLICATION

(11) **EP 1 526 136 A1**
(43) Date of publication of application: **27.04.2005**
(21) Application number: 04078477.9
(22) Date of filing: 02.08.2001
(51) Int. Cl.: C07D 487/04, A61K 31/519, A61P 25/08, A61P 25/22, A61P 21/02, A61P 25/20

(54) **Polymorphs of zaleplon and methods for the preparation thereof**

(30) Priority: 03.08.2000 US 222785 P
(62) Divisional of application: 01961891.7
(71) Applicant: Wyeth, Madison, New Jersey 07940 (US)
(72) Inventor: Aslam, Farhan, Edgewater, NJ 07020 (US); Cowans, Brett, West Lafayette, IN 47906 (US); Byrn, Stephen R., West Lafayette, IN 47906 (US); Stahly, G. Patrick, West Lafayette, IN 47906 (US)
(74) Representative: Howard, Paul Nicholas

(57) **Abstract**

This invention relates to novel crystalline polymorphic forms of zaleplon (N-[3-(3-cyanopyrazolo[1,5a]pyrimidin-7-yl)phenyl]-N-ethylacetamide), methods for the preparation thereof, and their uses as anxiolytic, antiepileptic, and sedative-hypnotic agents and skeletal muscle relaxants.

## Description

This application claims the benefit of U.S. Provisional Application Serial No. 60/222,785, filed August 3, 2000, which is herein incorporated by reference.

### Field of the Invention

This invention relates to novel crystalline polymorphic forms of zaleplon (N-[3-(3-cyanopyrazolo[1,5a]pyrimidin-7-yl)phenyl]-N-ethylacetamide), methods for the preparation thereof, and their use as anxiolytic, antiepileptic, and sedative-hypnotic agents and skeletal muscle relaxants.

### Background of the Invention

Zaleplon is a generic term used to identify the chemical compound N-[3-(3-cyanopyrazolo[1,5a]pyrimidin-7-yl)phenyl]-N-etbylacetamide:

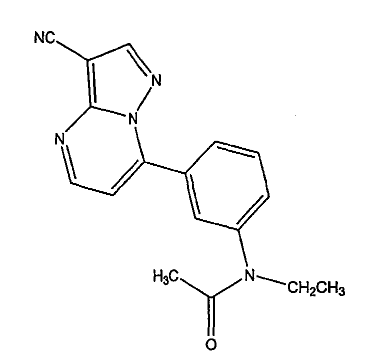

Syntheses for zaleplon are described in U.S. Patent Nos. 4,626,538 and 5,714,607, both of which are hereby incorporated by reference. Zaleplon is useful as an anxiolytic, antiepileptic, and sedative-hypnotic agent as well as a skeletal muscle relaxant.

### Summary of the Invention

The present inventors have discovered three novel crystalline polymorphs of zaleplon, referred hereinafter as Forms I, II, and III. Form I is an anhydrous crystal form, while Forms II and III are crystal forms which can be anhydrous or hydrates. These three forms of zaleplon, like other forms of the compound, are useful in the treatment of anxiety and epilepsy and to induce a sedative-hypnotic effect and relax skeletal muscles.

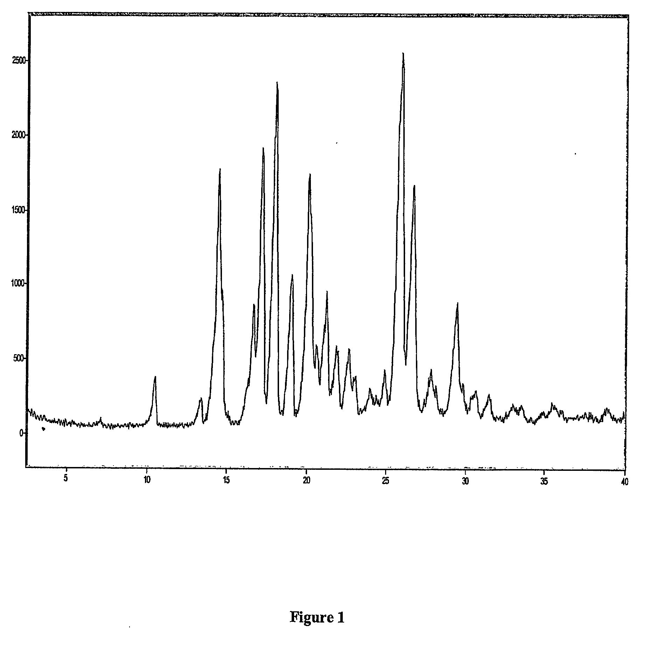

Form I has a melting point, as determined by differential scanning calorimetry (DSC), of from about 186 to about 189°C and exhibits a characteristic X-ray powder diffraction (XRPD) pattern with characteristic peaks (expressed in degrees 2θ ± 0.2° 2θ) at 10.4, 14.5, 16.7, 17.2, 18.0, 19.0, 20.1, 20.6, 21.2, 21.9, 22.6, 25.8, 26.6, 27.9, and 29.4 as depicted in Figure 1. In particular, the peaks (expressed in degrees 2θ ± 0.2° 2θ) at 10.4, 14.5, and 20.1 are unique to Form I.

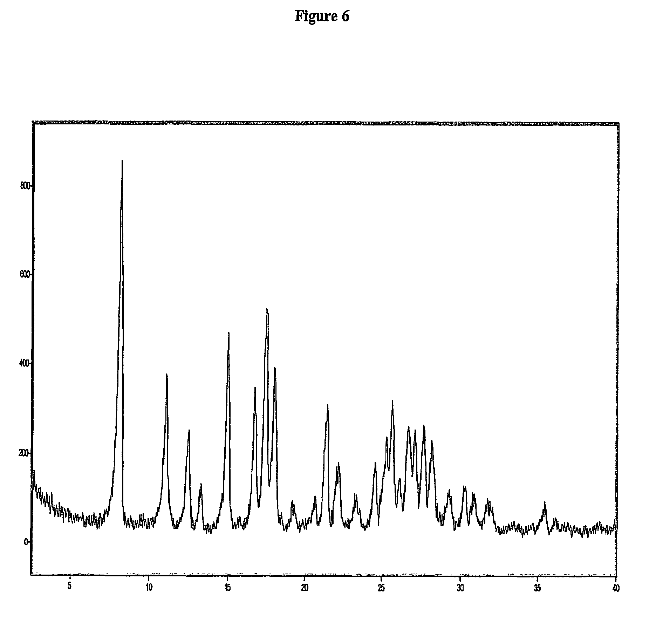

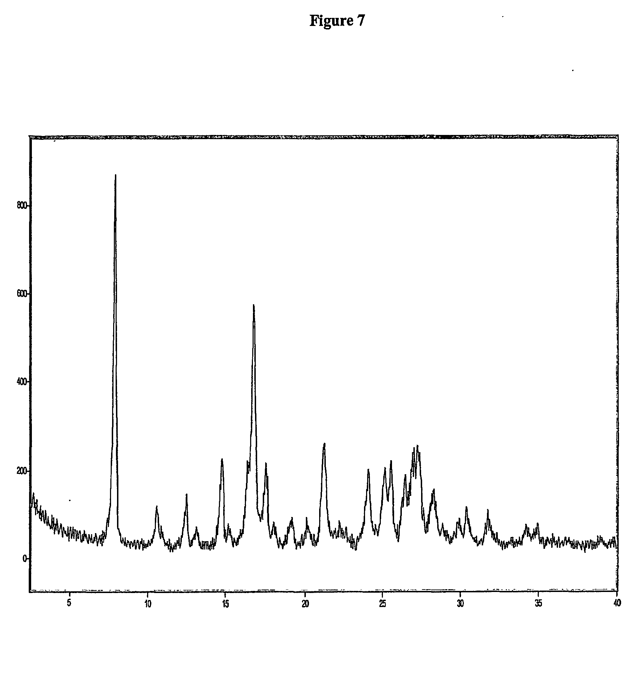

Form II exhibits a characteristic XRPD pattern with characteristic peaks (expressed in degrees 2θ ± 0.2° 2θ) at 7.9-8.1, 10.6-11.0, 12.5, 14.8-15.0, 16.8, 17.5-17.6, 21.2-21.4, 24.1-24.5, 25.1-25.2, 25.5-25.7, 27.0-27.1, 27.4-27.7, and 28.2-28.3 as depicted in Figures 6 and 7. In particular, the peaks (expressed in degrees 2θ ± 0.2° 2θ) at 12.5 and 21.2-21.4 are unique to Form II.

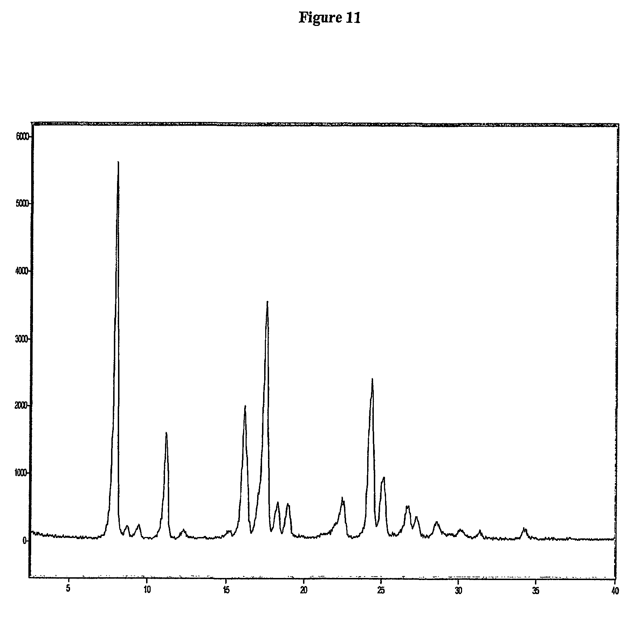

Form III exhibits a characteristic XRPD pattern with characteristic peaks (expressed in degrees 2θ ± 0.2° 2θ) at 8.0, 11.2, 16.2, 17.1, 17.6, 24.3, and 25.1 as depicted in Figure 11. In particular, the peak (expressed in degrees 2θ ± 0.2° 2θ) at 16.2 is unique to Form III.

Another embodiment is a pharmaceutical composition comprising one or more of Forms I, II, and III of zaleplon and, optionally, a pharmaceutically acceptable carrier or diluent. Typically, the pharmaceutical composition comprises an amount of one or more of Forms I, II, and III of zaleplon effective to treat anxiety or epilepsy or to induce a sedative-hypnotic effect or relax skeletal muscles in an animal, such as a mammal (*e.g.* human), and, optionally, a pharmaceutically acceptable carrier or diluent. According to one preferred embodiment, the pharmaceutical composition comprises at least about 20, 30, 40, 50, 60, 70, 80,90, 95, 96, 97, 98, 99, 99.1,99.2, 99.3,99.4,99.5,99.6, 99.7, 99.8, or 99.9% by weight of Form I of zaleplon, based upon 100% total weight of zaleplon in the pharmaceutical composition. According to another preferred embodiment, the pharmaceutical composition comprises at least about 20, 30, 40, 50, 60, 70, 80, 90, 95, 96, 97, 98, 99, 99.1, 99.2, 99.3, 99.4, 99.5, 99.6, 99.7, 99.8, or 99.9% by weight of Form II of zaleplon, based upon 100% total weight of zaleplon in the pharmaceutical composition. According to yet another preferred embodiment, the pharmaceutical composition comprises at least about 20, 30, 40, 50, 60, 70, 80, 90, 95, 96, 97, 98, 99, 99.1, 99.2, 99.3, 99.4, 99.5, 99.6, 99.7, 99.8, or 99.9% by weight of Form III of zaleplon, based upon 100% total weight of zaleplon in the pharmaceutical composition.

Yet another embodiment is a method of treating anxiety or epilepsy in an animal in need thereof by administering an anti-anxiety or anti-epilepsy effective amount of Form I, II, or III of zaleplon or a mixture thereof. Preferably, the zaleplon is administered orally.

Yet another embodiment is a method of inducing a sedative-hypnotic effect in an animal in need thereof by administering a sedative, hypnotic, or sedative and hypnotic effective amount of Form I, II, or III of zaleplon or a mixture thereof.

Yet another embodiment is a method of relaxing one or more skeletal muscles in an animal in need thereof by administering a skeletal muscle relaxing effective amount of Form I, II, or III of zaleplon or a mixture thereof.

Yet another embodiment is a method of preparing Form I of zaleplon by cooling zaleplon in a non-aqueous solvent, such as acetone and acetonitrile, from a temperature of 40°C or higher.

Another method of preparing Form I of zaleplon is by providing zaleplon in an organic solvent and evaporating the solvent at ambient temperature.

Yet another method of preparing Form I of zaleplon is by heating one or more of Forms II and III of zaleplon.

Yet another embodiment is a method of preparing Form II of zaleplon by crash precipitation of zaleplon with water. Crash precipitation can be performed by dissolving zaleplon in a non-aqueous solvent, such as an organic solvent, to form a solution and adding water to the solution.

Yet another embodiment is a method of preparing Form III of zaleplon by providing a solution containing zaleplon dissolved in an aqueous solvent and evaporating the solvent.

In each of the aforementioned methods of preparing crystalline polymorphs of zaleplon, the crystals formed may be recovered by any method known in the art.

### Brief Description of the Drawings

Figure 1 is a characteristic X-ray Powder Diffraction (XRPD) pattern for Form I of zaleplon.
Figure 2 is a ¹³C Solid State Nuclear Magnetic Resonance (SSNMR) spectrum of Form I of zaleplon.
Figure 3 is a moisture adsorption/desorption isotherm at 25° C of Form I of zaleplon.
Figure 4 is an ORTEP representation of the single crystal structure of Form I of zaleplon. Figure 5 is a calculated XRPD pattern for Form I of zaleplon.
Figure 6 is a characteristic XRPD pattern for Form II of zaleplon in a low moisture (approximately 20% relative humidity) environment.
Figure 7 is a characteristic XRPD pattern for Form II of zaleplon in a high moisture (approximately 95% relative humidity) environment.
Figure 8 is a moisture adsorption/desorption isotherm at 25°C of Form **II** of zaleplon.
Figure 9 is a SSNMR spectrum of Form II of zaleplon.

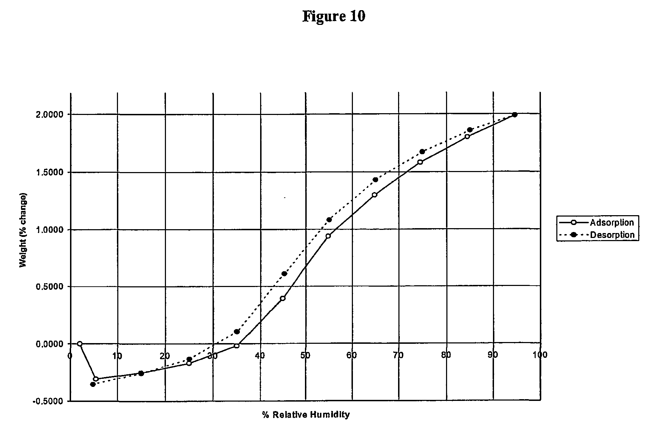

Figure 10 is a moisture adsorption/desorption isotherm at 25°C of Form III of zaleplon.
Figure 11 is a characteristic XRPD pattern for Form III of zaleplon.
Figure 12 is a SSNMR spectrum of Form III of zaleplon.

### Detailed Description of the Invention

Three novel crystalline polymorphs of zaleplon (herein referred to as Forms I, II, and III) have been discovered

### Form I of Zaleplon

Form I is an anhydrous crystalline form of zaleplon. Form I is most stable in the absence of water and is typically more stable than Forms II and III. Form I is stable under a broad range of humidity and temperature conditions. The term "anhydrous crystalline form" as used herein refers to a crystal form of zaleplon wherein each molecule of zaleplon in the crystal is not associated with water. Form I can be easily manufactured into a dosage unit form.

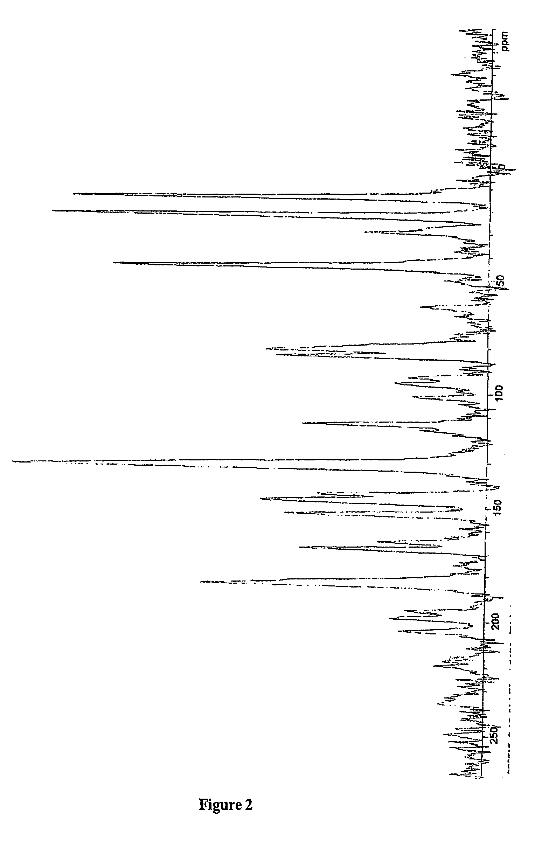

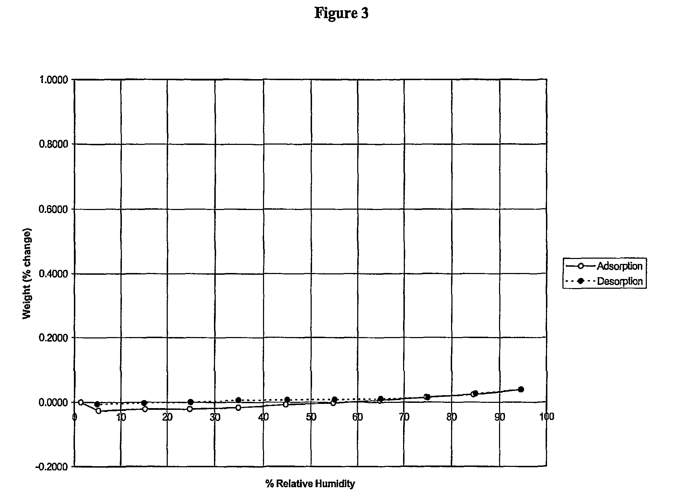

Form I has a distinct XRPD pattern and SSNMR spectrum as shown in Figures 1 and 2, respectively. Peak locations and relative intensities for the XRPD pattern of Form I are provided in Table 1 below. The peaks (expressed in degrees 2θ± 0.2° 2θ) at 10.4, 14.5, and 20.1 are unique to Form I. The chemical shifts and delta values for the lines in the SSNMR spectrum of Form I are provided in Table 9. The term "Form I" as used herein refers to crystalline polymorphs of zaleplon having this and substantially related XRPD patterns. Figure 3 shows the moisture adsorption/desorption curves for Form I. As shown by Figure 3, Form I of zaleplon is non-hygroscopic.

**Table 1**

| Characteristic XRPD Peaks (expressed in degrees 2θ± 0.2° 2θ) and Relative Intensities (>10) of Diffraction Lines for Form I of Zaleplon | | |
|---|---|---|
| Degrees 2θ (± 0.2° 2θ) | d (Å) | I/Io |
| 10.4 | 8.47 | 13 |
| 14.5 | 6.11 | 64 |
| 16.7 | 5.31 | 29 |
| 17.2 | 5.15 | 73 |
| 18.0 | 4.93 | 88 |
| 19.0 | 4.67 | 38 |
| 20.1 | 4.41 | 63 |
| 20.6 | 4.30 | 16 |
| 21.2 | 4.19 | 28 |
| 21.9 | 4.06 | 16 |
| 22.6 | 3.93 | 16 |
| 25.8 | 3.45 | 100 |
| 26.6 | 3.35 | 62 |
| 27.9 | 3.20 | 10 |
| 29.4 | 3.04 | 29 |

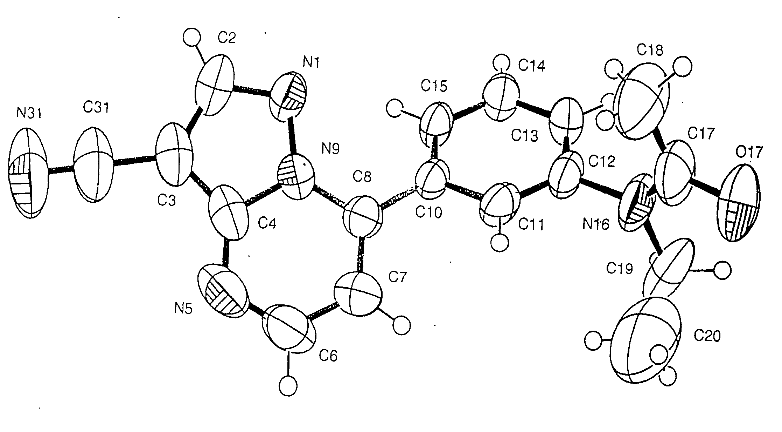

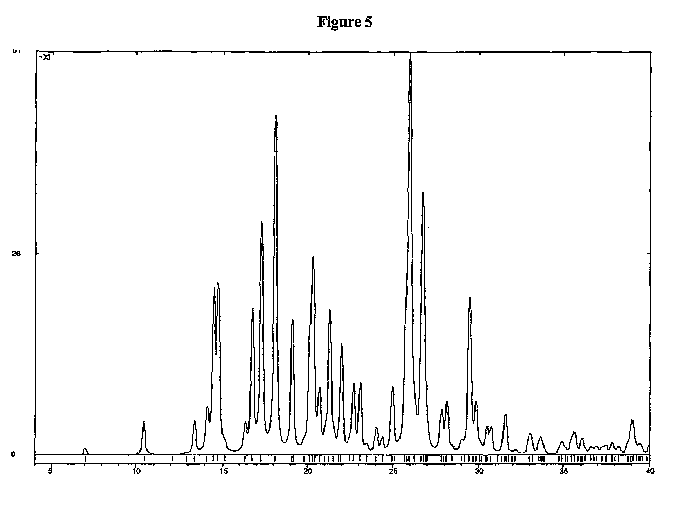

The crystal structure of Form I has been determined at 295 K. The unit cell parameters are shown in Table 2 and the atomic positions and temperature factors are shown in Tables 3, 4, and 5. The structure of Form I of zaleplon as drawn by ORTEP is shown in Figure 4. An XRPD pattern calculated from the data in Tables 2-5 is shown in Figure 5. The intensity differences between Figures 1 (experimental) and 5 (calculated) are due to preferred orientation. Forms I, II, and III have all been observed to exhibit patterns displaying preferred orientation effects.

**Table 2**

| Space Group and Unit Cell Parameters for Form I of Zalepon | |
|---|---|
| Parameter | Form I |
| Space group | P2₁/c (No. 14) |
| Cell dimensions | |
| *a* (Å) | 6.9760 (5) |
| *b* (Å) | 25.0623 (17) |
| *c* (Å) | 9.1369 (5) |
| β (°) | 100.92 (4) |
| Volume (Å³) | 1568.5 (5) |
| Z (Molecules/unit cell) | 4 |
| Density (g/cm³) | 1.293 |
| Data acquisition temperature | 295 K |

**Table 3**

| Atomic Coordinates and Isotropic Thermal Parameters (Å²) for Form I of Zaleplon | | | | |
|---|---|---|---|---|
| Atom | X | Y | Z | Uᵢₛₒ |
| 017 | 0.0660(7) | 0.0879(2) | 1.1382(4) | 0.1288(15) |
| N1 | 0.6206(4) | 0.12021(11) | 0.4154(3) | 0.0635(8) |
| N5 | 0.4122(6) | 0.24950(14) | 0.3361(5) | 0.0937(12) |
| N9 | 0.4851(4) | 0.15955(10) | 0.4183(3) | 0.0552(7) |
| N16 | 0.0599(5) | 0.0774(2) | 0.8965(3) | 0.0875(11) |
| N31 | 0.8544(8) | 0.2550(3) | 0.1277(6) | 0.157(3) |
| C2 | 0.7394(6) | 0.1411(2) | 0.3328(4) | 0.0775(11) |
| C3 | 0.6857(6) | 0.1927(2) | 0.2839(4) | 0.0775(11) |
| C4 | 0.5220(6) | 0.20448(15) | 0.3413(4) | 0.0718(10) |
| C6 | 0.2658(7) | 0.2465(2) | 0.4064(6) | 0.0935(14) |
| C7 | 0.2184(6) | 0.20206(15) | 0.4817(4) | 0.0767(10) |
| C8 | 0.3285(5) | 0.15681(12) | 0.4891(3) | 0.0549(7) |
| C10 | 0.2841(4) | 0.10815(11) | 0.5661(3) | 0.0488(7) |
| C11 | 0.2027(5) | 0.11412(14) | 0.6941(3) | 0.0598(8) |
| C12 | 0.1466(5) | 0.07044(15) | 0.7662(3) | 0.0622(9) |
| C13 | 0.1668(5) | 0.02016(15) | 0.7131(4) | 0.0666(9) |
| C14 | 0.2475(5) | 0.01345(14) | 0.5857(4) | 0.0644(9) |
| C15 | 0.3047(5) | 0.05695(12) | 0.5137(3) | 0.0547(7) |
| C17 | 0.1550(8) | 0.0814(2) | 1.0346(4) | 0.0928(14) |
| C18 | 0.3668(8) | 0.0774(3) | 1.0586(5) | 0.125(2) |
| C19 | -0.1644(11) | 0.0806(4) | 0.8635(9) | 0.153(3) |
| C20 | -0.2398(13) | 0.1254(6) | 0.8238(12) | 0.240(7) |
| C31 | 0.7792(7) | 0.2276(2) | 0.1979(6) | 0.108(2) |

**Table 4**

| H-Atom Coordinates and Isotropic Thermal Parameters (Å²) for Form I of Zaleplon | | | | |
|---|---|---|---|---|
| Atom | X | Y | Z | Uᵢₛₒ |
| H2 | 0.8469(6) | 0.1233(2) | 0.3101(4) | 0.101 |
| H6 | 0.1876(7) | 0.2766(2) | 0.4057(6) | 0.122 |
| H7 | 0.1104(6) | 0.20300(15) | 0.5277(4) | 0.1 |
| H11 | 0.1864(5) | 0.14812(14) | 0.7307(3) | 0.078 |
| H13 | 0.1271(5) | -0.00934(15) | 0.7614(4) | 0.087 |
| H14 | 0.2626(5) | -0.02067(14) | 0.5495(4) | 0.084 |
| H15 | 0.3580(5) | 0.05206(12) | 0.4288(3) | 0.071 |
| H18A | 0.4215(9) | 0.1120(4) | 1.0478(45) | 0.163 |
| H18B | 0.4033(8) | 0.0534(13) | 0.9866(30) | 0.163 |
| H18C | 0.4154(9) | 0.0641(16) | 1.1572(17) | 0.163 |
| H19A | -0.2128(11) | 0.0549(4) | 0.7857(9) | 0.198 |
| H19B | -0.2109(11) | 0.0694(4) | 0.9523(9) | 0.198 |
| H20A | -0.3795(14) | 0.1222(11) | 0.8033(110) | 0.313 |
| H20B | -0.1962(119) | 0.1372(20) | 0.7356(66) | 0.313 |
| H20C | -0.2010(120) | 0.1509(12) | 0.9022(46) | 0.313 |

**Table 5**

| Anisotropic Thermal Parameters (Å²) for Zaleplon Form I | | | | | | |
|---|---|---|---|---|---|---|
| Atom | U₁₁ | U₂₂ | U₃₃ | U₂₃ | U₁₃ | U₁₂ |
| 017 | 0.156(3) | 0.172(4) | 0.078(2) | 0.000(2) | 0.072(2) | 0.004(3) |
| N1 | 0.066(2) | 0.063(2) | 0.069(2) | -0.0058(12) | 0.0335(13) | -0.0065(12) |
| N5 | 0.099(3) | 0.071(2) | 0.105(3) | 0.035(2) | 0.003(2) | -0.003(2) |
| N9 | 0.066(2) | 0.0512(13) | 0.0507(13) | 0.0011(10) | 0.0161(11) | -0.0058(11) |
| N16 | 0.072(2) | 0.143(3) | 0.057(2) | 0.005(2) | 0.0364(15) | 0.009(2) |
| N31 | 0.127(4) | 0.217(6) | 0.128(4) | 0.074(4) | 0.021(3) | -0.075(4) |
| C2 | 0.075(2) | 0.092(3) | 0.075(2) | -0.013(2) | 0.038(2) | -0.025(2) |
| C3 | 0.082(2) | 0.089(3) | 0.063(2) | 0.009(2) | 0.017(2) | -0.033(2) |
| C4 | 0.080(2) | 0.070(2) | 0.062(2) | 0.017(2) | 0.005(2) | -0.021(2) |
| C6 | 0.094(3) | 0.067(2) | 0.118(4) | 0.028(2) | 0.014(3) | 0.013(2) |
| C7 | 0.082(2) | 0.068(2) | 0.081(2) | 0.009(2) | 0.020(2) | 0.016(2) |
| C8 | 0.060(2) | 0.057(2) | 0.0492(15) | -0.0003(12) | 0.0135(13) | 0.0039(13) |
| C10 | 0.0475(14) | 0.058(2) | 0.0443(13) | -0.0012(11) | 0.0167(11) | 0.0023(12) |
| C11 | 0.060(2) | 0.075(2) | 0.049(2) | -0.0062(14) | 0.0210(13) | 0.0114(15) |
| C12 | 0.052(2) | 0.092(2) | 0.047(2) | 0.0029(15) | 0.0213(13) | 0.002(2) |
| C13 | 0.063(2) | 0.077(2) | 0.065(2) | 0.016(2) | 0.026(2) | -0.002(2) |
| C14 | 0.070(2) | 0.062(2) | 0.067(2) | -0.0003(15) | 0.028(2) | -0.0056(15) |
| C15 | 0.061(2) | 0.061(2) | 0.0489(15) | -0.0040(12) | 0.0264(13) | -0.0013(13) |
| C17 | 0.107(3) | 0.125(4) | 0.055(2) | 0.000(2) | 0.038(2) | -0.002(3) |
| C18 | 0.096(4) | 0.218(7) | 0.062(2) | -0.012(3) | 0.013(2) | -0.006(4) |
| C19 | 0.125(5) | 0.212(8) | 0.150(6) | 0.003(5) | 0.101(5) | 0.034(5) |
| C20 | 0.118(6) | 0.446(22) | 0.158(8) | 0.064(11) | 0.028(6) | 0.070(9) |
| C31 | 0.093(3) | 0.144(4) | 0.087(3) | 0.029(3) | 0.016(2) | -0.051(3) |

One method of preparing Form I of zaleplon is by cooling zapelon in a non-aqueous solvent. Preferably, the zaleplon is slowly cooled. For example, Form I of zaleplon can be formed by dissolving zaleplon in a non-aqueous solvent, heating it to at least about 40°C, and cooling it (e.g. to ambient temperature). Suitable non-aqueous solvents include, but are not limited to, organic solvents, such as acetone, acetonitrile, tetrahydrofuran (THF), methanol, and isopropanol. The solution is preferably heated to from about 50 to about 70 ° C, and more preferably to about 60° C. According to one embodiment, cooling occurs for about 4 to about 10 hours and more preferably about 6 hours.

Form I of zaleplon may also be prepared by evaporation crystallization methods, such as slow and fast evaporation crystallization methods, as known in the art. One preferred method of fast evaporation involves (i) dissolving zaleplon in a non-aqueous solvent, and (ii) removing the solvent from the solution quickly, such as by vacuum. Suitable non-aqueous solvents include, but are not limited to, organic solvents, such as acetone, dimethylformamide, ethylacetate, isopropanol, and tetrahydrofuran.

One preferred method of slow evaporation involves (i) dissolving zaleplon in a non-aqueous solvent at room temperature and (ii) incubating the mixture at room temperature to allow evaporation to occur slowly. Typically, evaporation occurs over a period of time of from about 12 to about 24 hours or longer. Suitable non-aqueous solvents include, but are not limited to, organic solvents, such as , acetone, acetonitrile, dimethylformamide, ethylacetate, and tetrahydrofuran.

Form I may also be prepared by heating one or more of Forms II and III of zaleplon to remove the water therein and recrystallize it. For example, Form I can be formed by heating Form II or III of zaleplon at a temperature of at least 60°C and preferably at a temperature of at least about 75 or 80°C.

The crystals formed may be recovered by any method known in the art, such as filtration, centrifugation, or with a Buchner style filter, Rosenmund filter, or plates and frame press. Typically, the crystals are recovered as solids.

### Form II of Zaleplon

Form II is a variable-water hydrate crystalline form of zaleplon, i.e., the number of water molecules associated with each molecule of zaleplon may vary. The term "hydrate" refers to a crystal form of zaleplon wherein at least one molecule of zaleplon in the crystal is associated with water. The number of water molecules associated with each molecule of zaleplon can vary from 0 to about 1, i.e. Form II can be anhydrous or a hydrate. The term "variable-water hydrate" includes both anhydrous and hydrate forms of the polymorph. For example, Form II can be a monohydrate or hemihydrate of zaleplon. The term "monohydrate" as used herein refers to a hydrate in which one molecule of water is associated with each molecule of zaleplon. The term "hemihydrate" as used herein refers to a hydrate in which one molecule of water is associated with two molecules of zaleplon. The inventors have found that while Form II is stable at about 40°C and about 75% relative humidity for 4 weeks, Form II converts into Form I when stored at about 60°C and about 75% relative humidity over the same time period. Form II also converts into Form I when heated at about 80°C Form II of zaleplon is particularly suitable for immediate or rapid release formulations.

The crystal structure of Form II has been determined at 150 K and is shown in Table 6 below. At 150 K, Form II of zaleplon is a hemihydrate. The XRPD pattern of Form II of zaleplon varies slightly with its moisture content. Two XRPD patterns of Form II of zaleplon at different relative humidity are shown in Figures 6 (low moisture, approximately 20% relative humidity) and 7 (high moisture, approximately 95% relative humidity). The characteristic peak positions and relative intensities for the XRPD patterns in Figures 6 and 7 are shown in Table 7. The peaks (expressed in degrees 2θ± 0.2° 2θ) at 12.5 and 21.4 are unique to Form II at approximately 20% relative humidity and at 12.5 and 21.2 are unique to Form II at approximately 95% relative humidity. Generally, the peaks (expressed in degrees 2θ± 0.2° 2θ) at 12.5 and 21.2-21.4 are unique to Form II. The term "Form II" as used herein refers to crystalline polymorphs of zaleplon having these and substantially related XRPD patterns.

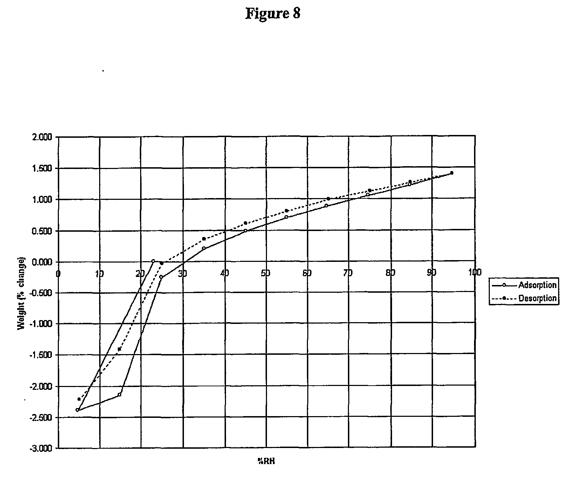

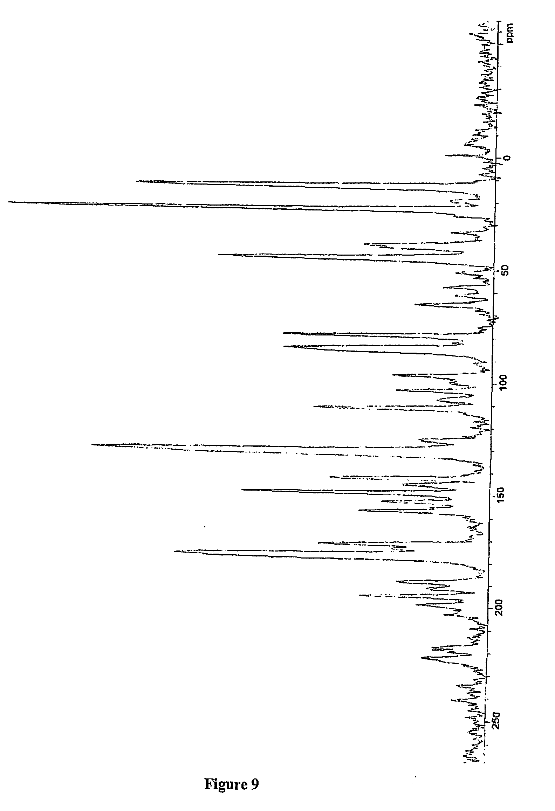

Figure 8 shows moisture adsorption/desorption curves for Form II of zaleplon. It is clear from Figure 8 that the moisture content of Form II of zaleplon varies depending on the relative humidity of its environment. Form II is more soluble in water than Form III and thus is more desirable for dosage unit forms when faster release rates are desired. Form II also exhibits a distinct SSNMR spectrum as shown in Figure 9. The chemical shifts and delta values for the lines in the SSNMR spectrum of Form II shown in Figure 9 are provided in Table 9.

**Table 6**

| Space Group and Unit Cell Parameters for Zaleplon Form II | |
|---|---|
| Parameter | Form II |
| Space group | P2₁/c (No. 14) |
| Cell Dimensions | |
| *a* (Å) | 11.1896 (9) |
| *b* (Å) | 6.9236 (5) |
| *c* (Å) | 20.986 (2) |
| β(°) | 99.089 (3) |
| Volume (Å³) | 1605.4 (4) |
| Z (Molecules/unit cell) | 4 |
| Density (g/cm³) | 1.300 |
| Data acquisition temperature | 150 K |

**Table 7**

| Characteristic XRPD Peaks (expressed in degrees 2θ ± 0.2° 2θ) and Relative Intensities (>10) of Diffraction Lines for Form II of Zaleplon | | | | | |
|---|---|---|---|---|---|
| Low Moisture Content (Approximately 20% Relative Humidity) | | | High Moisture Content (Approximately 95% Relative Humidity) | | |
| Degrees 2θ (±0.2°26θ) | d (Å) | I/Io | Degrees 2θ (±0.2°26) | d (Å) | I/Io |
| 8.1 | 10.89 | 100 | 7.9 | 11.17 | 100 |
| 11.0 | 8.01 | 41 | 10.6 | 8.31 | 10 |
| 12.5 | 7.09 | 27 | 12.5 | 7.10 | 11 |
| 13.3 | 6.66 | 11 | - | - | - |
| 15.0 | 5.91 | 53 | 14.8 | 6.00 | 24 |
| - | - | - | 16.4 | 5.40 | 20 |
| 16.8 | 5.28 | 38 | 16.8 | 5.28 | 63 |
| 17.5 | 5.07 | 61 | 17.6 | 5.05 | 21 |
| 18.0 | 4.92 | 43 | - | - | - |
| 21.4 | 4.14 | 32 | 21.2 | 4.18 | 26 |
| 22.2 | 4.00 | 15 | - | - | - |
| - | - | - | 23.9 | 3.71 | 12 |
| 24.5 | 3.62 | 15 | 24.1 | 3.69 | 18 |
| 25.1 | 3.54 | 10 | 25.2 | 3.54 | 17 |
| 25.3 | 3.51 | 21 | - | - | - |
| 25.7 | 3.47 | 31 | 25.5 | 3.49 | 19 |
| - | - | - | 26.4 | 3.37 | 15 |
| 26.7 | 3.33 | 23 | - | - | - |
| 27.1 | 3.29 | 23 | 27.0 | 3.30 | 20 |
| - | - | - | 27.2 | 3.27 | 23 |
| 27.7 | 3.22 | 24 | 27.4 | 3.25 | 21 |
| 28.2 | 3.16 | 19 | 28.3 | 3.16 | 10 |
| 30.3 | 2.95 | 11 | - | - | - |

Form II of zaleplon may be prepared by crash precipitation of zaleplon. According to one preferred embodiment, crash precipitation includes dissolving zaleplon in a non-aqueous solvent, such as an organic solvent, at room temperature. Suitable organic solvents include, but are not limited to, acetone and tetrahydrofuran. The resulting solution is slowly added to water to form a precipitate. The crystals may be recovered by any method known in the art, including, but not limited to, those discussed above.

Typically, Form II converts into Form III in a solvent system containing an organic solvent and optionally, water. Form II can also be converted into Form III in water.

### Form III of Zaleplon

Form III is also a variable-water hydrate crystalline form of zaleplon. Form III is generally more stable in aqueous and non-aqueous environments than Form II. The number of water molecules associated with each molecule of zaleplon can vary from 0 to about 0.5, i.e. Form III can be anhydrous or a hydrate. For example, Form III can be a hemihydrate of zaleplon. Form III is generally anhydrous up to a relative humidity of about 30%. Also, hydrates of Form III can convert to Form II, e.g. by storing them at about 40°C and about 75% relative humidity, resulting in a mixture of Forms II and III. When Form III is stored at about 60° C and about 75% relative humidity or heated to about 80° C, it converts to Form I.

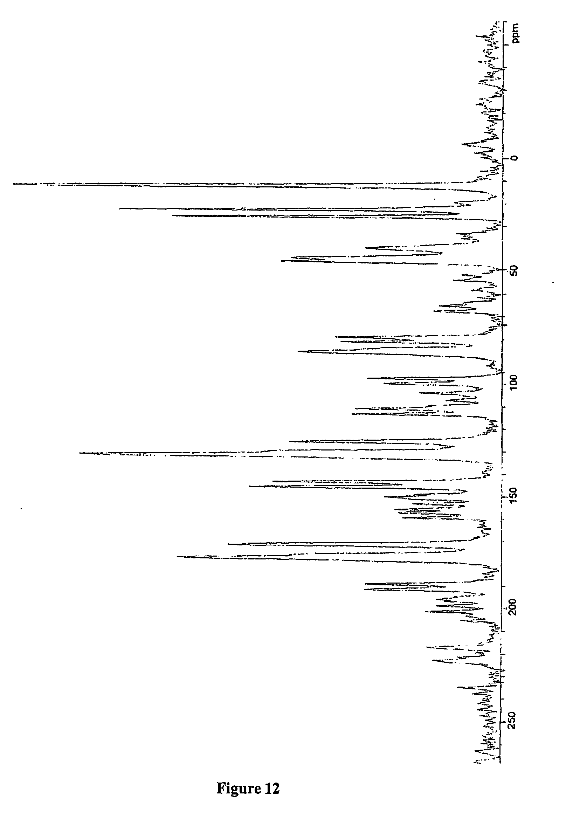

Form III has a distinct XRPD pattern and SSNMR spectrum as shown in Figures 11 and 12, respectively. The characteristic peak positions and relative intensities for the XRPD pattern in Figure 11 are provided in Table 8. The chemical shifts and delta values for the lines in the SSNMR spectrum of Form III are provided in Table 9.

**Table 8**

| Characteristic XRPD Peaks (expressed in degrees 2θ ± 0.2° 2θ) and Relative Intensities (>10) of Diffraction Lines for Form III of Zaleplon | | |
|---|---|---|
| Degrees 2θ (± 0.2° 2θ) | d (Å) | I/Io |
| 8.0 | 11.02 | 100 |
| 11.2 | 7.91 | 28 |
| 16.2 | 5.47 | 34 |
| 17.1 | 5.17 | 10 |
| 17.6 | 5.04 | 62 |
| 24.3 | 3.65 | 42 |
| 25.1 | 3.55 | 16 |

**Table 9**

| ¹³C Solid-State NMR (SSNMR) Chemical Shifts of Zaleplon | | | | | | |
|---|---|---|---|---|---|---|
| Carbon Atom | Form I | | Form II | | Form III | |
| | C.S.^{a} | Delta^{b} | C.S.^{a} | Delta^{b} | C.S.^{a} | Delta ^{b} |
| CH₃ | 14.3 | REF | 13.2 | REF | 12.1 & 12.4 | REF & 0.3 |
| CH₃ | 21.9 | 7.6 | 23.6 | 10.4 | 22.8 & 25.8 | 10.7 & 13.7 |
| CH₂ | 44.2 | 29.9 | 44.9 | 31.7 | 44.1 & 45.5 | 32.0 & 33.4 |
| Aromatic C or CN | 83.5 | 69.2 | 79.0 | 65.8 | 79.0 & 81.1 | 66.9 & 69.0 |
| Aromatic C or CN | 113.3 | 99.0 | 111.3 | 98.1 | 111.0 & 113.4 | 98.9 & 101.3 |
| Aromatic C | 132.2 | 117.9 | 130.7 | 117.5 | 131.4 | 119.3 |
| Aromatic C | 143.9 & 146.6 | 129.6 & 132.3 | 142.7 & 145.3 | 129.5 & 132.1 | 143.3 & 145.7 | 131.2 & 133.6 |
| Aromatic C | 152.7 | 138.4 | 149.3 & 153.1 | 136.1 & 139.9 | 149.0, 150.1, 153.0, & 155.5 | 136.9, 138.0, 140.9, & 143.4 |
| CO | 167.8 | 153.5 | 171.7 & 173.8 | 158.5 & 160.6 | 171.6 | 159.5 |

| | | | | | | |
|---|---|---|---|---|---|---|
| ^{a} - Chemical Shift (C.S.) in parts per million (± 0.2 ppm) relative to adamantane external standard. | | | | | | |
| ^{b} - Delta is the difference between the reference (REF) and selected peak in parts per million (ppm). | | | | | | |

Form III of zaleplon may be prepared by forming a solution containing zaleplon dissolved in an aqueous solvent and evaporating the solvent from the solution. Suitable solvents include, but are not limited to, mixtures of water with acetone, acetonitrile, or tetrahydrofuran (THF). Preferred solvents include, but are not limited to, mixtures of water with acetone, acetonitrile, or THF having a volume ratio of from about 1:1 to about 1:2. The resulting crystals may be recovered by any method known in the art, including, but not limited to, those discussed above.

Form III may also be prepared by dissolving Form II in a solvent system containing an organic solvent (such as those discussed above), water or a mixture thereof.

The aforementioned crystalline polymorphs of zaleplon are useful anxiolytics, antiepileptics, and sedative-hypnotic agents as well as skeletal muscle relaxants. The appropriate dosage amounts for an animal can be determined by methods known in the art. Generally, a therapeutic effective amount for the desired purpose is administered. The individual dosage of the crystalline polymorphs of zaleplon disclosed herein can be from about 5 to about 20 mg and preferably is from about 10 to about 20 mg for an adult.

These crystalline polymorphs can be formulated into a pharmaceutical composition. Preferably, the pharmaceutical composition comprises an amount of one or more of Forms I, II, and III of zaleplon effective to treat anxiety or epilepsy or to induce a sedative-hypnotic effect or relax skeletal muscles in an animal, such as a human. The term "sedative-hypnotic effect" refers to sedative effects, hypnotic effects, and sedative and hypnotic effects. According to one preferred embodiment, the pharmaceutical composition comprises at least about 20, 30, 40, 50, 60, 70, 80, 90, 95, 96, 97,98,99,99.1,99.2,99.3, 99.4, 99.5, 99.6, 99.7, 99.8, or 99.9% by weight of Form I of zaleplon, based upon 100% total weight of zaleplon in the pharmaceutical composition. According to another preferred embodiment, the pharmaceutical composition comprises at least about 20, 30, 40, 50, 60, 70, 80, 90, 95, 96, 97, 98, 99, 99.1, 99.2, 99.3, 99.4, 99.5, 99.6, 99.7, 99.8, or 99.9% by weight of Form II of zaleplon, based upon 100% total weight of zaleplon in the pharmaceutical composition. According to yet another preferred embodiment, the pharmaceutical composition comprises at least about 20, 30, 40, 50, 60, 70, 80, 90, 95, 96, 97, 98, 99, 99.1, 99.2, 99.3, 99.4, 99.5, 99.6, 99.7, 99.8, or 99.9% by weight of Form III of zaleplon, based upon 100% total weight of zaleplon in the pharmaceutical composition.

According to yet another preferred embodiment, the pharmaceutical composition comprises at least about 20, 30, 40, 50, 60, 70, 80, 90, 95, 96, 97, 98, 99, 99.1, 99.2, 99.3, 99.4, 99.5, 99.6, 99.7, 99.8, or 99.9% by weight of Form I of zaleplon, based upon 100% total weight of crystalline zaleplon in the pharmaceutical composition. According to yet another preferred embodiment, the pharmaceutical composition comprises at least about 20, 30, 40, 50, 60, 70, 80, 90, 95, 96, 97, 98, 99, 99.1, 99.2, 99.3, 99.4, 99.5, 99.6, 99.7, 99.8, or 99.9% by weight of Form II of crystalline zaleplon, based upon 100% total weight of zaleplon in the pharmaceutical composition. According to yet another preferred embodiment, the pharmaceutical composition comprises at least about 20, 30, 40, 50, 60, 70, 80, 90, 95, 96, 97, 98, 99, 99.1, 99.2, 99.3, 99.4, 99.5, 99.6, 99.7, 99.8, or 99.9% by weight of Form III of zaleplon, based upon 100% total weight of crystalline zaleplon in the pharmaceutical composition.

The pharmaceutical composition can also be substantially free or completely free of one or two of Forms I, II, and III of zaleplon as long as it contains at least one of Forms I, II, and TII. The term "substantially free" includes those pharmaceutical compositions that contain less than 0.01,0.1,0.2, 0.3, 0.4, 0.5, 1 or 2% by weight of one or more of Forms I, II, and III, based upon the total weight of pharmaceutical composition (or alternatively based upon on the total weight of zaleplon in the pharmaceutical composition).

The pharmaceutical composition broadly contains from about 1 to about 40 mg, preferably from about 5 to about 20 mg, and more preferably from about 5 to about 10 mg of one or more of Forms I, II, and III of zaleplon.

Generally, the pharmaceutical composition also includes one or more pharmaceutically acceptable carriers or diluents and excipients. The term "excipient" includes, but is not limited to, those materials that are acceptable for use in pharmaceutical formulations, and are added to the formulation to promote the stability and viability of the formulation, such as binders, bulking agents, clarifying agents, buffering agents, wetting agents, and lubricants including, but not limited to starch, pregelatinized starch, lactose, mannitol, methyl cellulose, microcrystalline cellulose, talc, highly dispersed silcic acids, silicon dioxide, high molecular weight fatty acids (such as stearic acid), gelatine agaragar, calcium phosphate, magnesium stearate, animal and vegetable fats and solid high molecular weight polymers (such as polyethylene glycol), sweeteners and or flavoring agents. Suitable pharmaceutically acceptable carriers, diluents, and excipients also include those described in Remington's, The Science and Practice of Pharmacy, (Gennaro, A.R., ed., 19^{th} edition, 1995, Mack Pub. Co.) which is herein incorporated by reference. The phrase "pharmaceutically acceptable" refers to additives or compositions that are physiologically tolerable and do not typically produce an allergic or similar untoward reaction, such as gastric upset, dizziness and the like, when administered to an animal, such as a mammal (e.g. a human).

The pharmaceutical composition may be a dosage form, such as a liquid (e.g. an aqueous solution containing Forms II and/or III of zaleplon or a non-aqueous solution containing Form I of zaleplon), capsule, pill, or tablet The pharmaceutical compositions and the crystalline polymorphs of zaleplon may be administered to animals, including, but not limited to, mammals (e.g. humans), orally, intravenously, parenterally, intramuscularly, or subcutaneously. Preferably, the composition is administered orally.

### Methods of Characterization

### 1. X-Ray Powder Diffraction

X-ray powder diffraction analyses were carried out on a Shimadzu XRD-6000 X-ray powder diffractometer, available from Shimadzu Scientific Instruments, Inc. of Columbia, MD, using Cu *Kα* radiation. The instrument was equipped with a fine-focus X-ray tube. The tube power was set to 40 kV and 40 mA. The divergence and scattering slits were set at 1° and the receiving slit was set at 0.15 mm. Diffracted radiation was detected by a NaI scintillation detector. A theta-two theta continuous scan at 3°/min (0.4 sec/0.02° step) from 2.5 to 40° 2θ was used. A silicon standard was analyzed each day to check the instrument alignment. Each sample was prepared for analysis by filling a low background quartz or silicon sample holder.

### 2. ¹³C Solid State NMR (SSNMR) Spectroscopy

Solid-state ¹³C NMR data were obtained with a 360 MHz Tecmag spectrometer, available from Tecmag, Inc. of Houston, TX. High resolution spectra were obtained with high-power proton decoupling and cross polarization with magic angle spinning at approximately 4 to 5 kHz. Approximately 150 to 200 mg of each sample was packed into a zirconia rotor. Data were collected at a ¹³C resonance frequency of 91.369 MHz, with a 30 kHz sweep width / filter, 1K data points, and 700 to 800 acquisitions. Additional parameters included a 7 µs ¹H pulse width and a 20 second pulse delay. The FID data was processed by zerofilling to 4K data points and multiplying by 20 Hz exponential line broadening prior to Fourier transformation. The chemical shifts were referenced externally to adamantane.

### 3. Moisture Balance

Moisture adsorption / desorption data were collected on a VTI SGA-100 moisture balance system, available from VTI Corporation of Hialeah, FL. For adsorption isotherms, an adsorption range of 5 to 95% relative humidity and a desorption range of 95 to 5% relative humidity in 10% relative humidity increments were used for analysis. The samples were not dried prior to analysis. Equilibrium criteria used for analysis were less than 0.0100 weight percent change in 5 minutes with a maximum equilibration time of 3 hours if the weight criterion was not met. Data were not corrected for the initial moisture content of the samples.

### 4. X-ray Single Crystal Structure Determination

A single crystal of Form I or Form II of zaleplon was mounted on a glass fiber in a random orientation. Preliminary examination and data collection were performed with Cu or Mo *Kα* radiation on a Enraf-Nonius CAD4 or a Nonius KappaCCD, available from Bruker Nonius B.V. of Delft, The Netherlands. The crystallographic drawing was obtained using the program ORTEP. The space group was determined using the program ABSEN. The structure was solved by direct methods. The remaining atoms were located in succeeding difference Fourier syntheses. Hydrogen atoms were included in the refinement but restrained to ride on the atom to which they are bonded.

### EXAMPLES

The following examples are illustrative and are not meant to limit the scope of the claimed invention. Zaleplon in the following examples can be prepared as described in U.S. Patent Nos. 4,626,538 and 5,714,607.

### Example 1

### Preparation of Form I of Zaleplon

Excess zaleplon is dissolved in acetone. The mixture was heated on a heating plate with stirring at 60 ° C and filtered through a 0.2 micron Teflon filter into an Erlenmeyer flask in a water bath at 60°C. The flask was incubated at room temperature for 24 hours. Crystals were recovered by filtration and allowed to dry for 24 hours at room temperature.

### Example 2

### Preparation of Form I of Zaleplon

The procedure described in Example 1 was repeated substituting acetonitrile for acetone.

### Example 3

### Preparation of Form II of Zaleylon

Approximately 5 g of zaleplon of Form I was dissolved in 125 ml of tetrahydrofuran (THF) in 10 ml aliquots with sonication. The clear solution was filtered through a 0.2 micron nylon filter into 700 ml of water at approximately 3°C with stirring. A precipitate formed immediately. The precipitate was filtered and dried in air at ambient temperature.

### Example 4

### Preparation of Form II of Zaleplon

Zaleplon of Form I was dissolved in either acetone or THF to yield a saturated solution. The solution was slowly poured into a dry-ice cooled slurry of water to yield a solution having a volume ratio of acetone to water or THF to water of about 2.9:1. Precipitation occurred during this process. The solution with the solids was left at ambient temperature for about 2 hours. The solids were collected by suction filtration and air-dried at room temperature.

### Example 5

### Preparation of Form II of Zaleplon

Approximately 30 mg of zaleplon of Form I was dissolved in approximately 1.2 ml of acetone with sonication. The solution was filtered to yield a clear solution. The solution was allowed to evaporate under ambient conditions to produce solids.

### Example 6

### Preparation of Form III of Zaleplon

Approximately 5.5 g of zaleplon of Form I was dissolved in approximately 145 ml of THF in 10 ml aliquots with sonication. The solution was filtered through a 0.2 micron nylon filter to yield a clear solution. Approximately 290 ml of water was added slowly to the solution with stirring at room temperature. The solution was allowed to evaporate under ambient conditions. After approximately 6 days, a small amount of solution and a large amount of solid remained. The solution was filtered and the recovered solid was dried in air at ambient temperature.

### Example 7

### Preparation of Form III of Zaleplon

Approximately 0.5 g of zaleplon of Form I was dissolved in 3.6 ml of THF and water solution having a volume ratio of about 1:2 (THF:water) with sonication. The slurry was agitated for 14 days at ambient temperature. The solids remaining were filtered and dried in air at ambient temperature.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

It is further to be understood that values are approximate, and are provided for description.

Patents, patent applications, publications, procedures, and the like are cited throughout this application, the disclosures of which are incorporated herein by reference in their entireties. To the extent that a conflict may exist between the specification and a reference, the language of the disclosure made herein controls.

## Claims

1. Crystalline polymorph Form II of N-[3-(3-cyanopyrazolo[1,5a]pyrimidin-7yl) phenyl]-N-ethylacetamide.

2. A variable-water hydrate crystalline polymorph of N-[3-(3cyanopyrazolo[1,5a]pyrimidin-7-yl) phenyl]-N-ethylacetamide.

3. The crystalline polymorph of claim 2, wherein the polymorph is a hydrate.

4. The crystalline polymorph of claim 2, wherein the crystalline polymorph exhibits an X-ray powder diffraction pattern having characteristic peaks expressed in degrees 2θ at about 12.5 and 21.4 ± 0. 2 °2θ.

5. The crystalline polymorph of claim 2, wherein the crystalline polymorph exhibits an X-ray powder diffraction pattern having characteristic peaks expressed in degrees 2θ at about 12.5 and 21.2 ± 0.2 °2θ.

6. The crystalline polymorph of claim 2, wherein the crystalline polymorph exhibits an X-ray powder diffraction pattern having characteristic peaks expressed in degrees 2θ at about 8. 1, 11.0, 12.5, 13.3, 15.0, 16.8, 17.5, 18.0, 21.4, 22.2, 24.5, 25.1, 25.3, 25.7, 26.7, 27.1, 27.7, 28.2, and 30.3 ± 0.2 °2θ.

7. The crystalline polymorph of claim 2, wherein the crystalline polymorph exhibits an X-ray powder diffraction pattern having characteristic peaks expressed in degrees 2θ at about 7.9, 10.6, 12.5, 14.8, 16.4, 16.8, 17.6, 21.2, 23.9, 24.1, 25.2, 25.5, 26.4, 27.0, 27.2, 27.4, and 28.3 ± 0.2 °2θ.

8. The crystalline polymorph of claim 2, wherein the crystalline polymorph exhibits an X-ray powder diffraction pattern substantially the same as that shown in Figure 6.

9. The crystalline polymorph of claim 2, wherein the crystalline polymorph exhibits an X-ray powder diffraction pattern substantially the same as that shown in Figure 7.

10. The crystalline polymorph of claim 2, wherein the crystalline polymorph exhibits a chemical shift in a ¹³C Solid State Nuclear Magnetic Resonance spectrum at about 13.1 and 23. 6 ± 0.2 ppm.

11. The crystalline polymorph of claim 10, wherein the crystalline polymorph exhibits chemical shifts in a ¹³C Solid State Nuclear Magnetic Resonance spectrum at about 13.2, 23.6, 44.9, 79.0, 111.3, 130.7, 142.7, 145.3, 149.3, 153.1, 171.7, and 173. 8 ± 0.2 ppm.

12. The crystalline polymorph of claim 2, wherein the crystalline polymorph exhibits a difference between the lowest ppm peak and another peak in a ¹³C Solid State Nuclear Magnetic Resonance spectrum of about 10.4 ppm.

13. The crystalline polymorph of claim 12, wherein the crystalline polymorph exhibits delta values in a ¹³C Solid State Nuclear Magnetic Resonance spectrum of about 10.4, 31.7, 65.8, 98.1, 117.5, 129.5, 132.1, 136.1, 139.9, 158.5, and 160.6 ppm.

14. The crystalline polymorph of claim 2, wherein the crystalline polymorph exhibits an X-ray powder diffraction pattern substantially the same as that shown in Figure 9.
| Parameter | Form II |
|---|---|
| Space group | P2₁/_{C} (No. 14) |
| Cell Dimensions | |
| *a* (Å) | 11.1896(9) |
| *b* (Å) | 6-9236(5) |
| *c* (Å) | 20.986 (2) |
| β (°) | 99.089(3) |
| Volume (Å³) | 1605.4 (4) |
| Z (Molecules/unit cell) | 4 |
| Density (g/cm³) | 1.300 |

15. A crystalline polymorph of N-[3-(3-cyanopyrazolo[1,5a]pyrimidin-7-yl) phenyl]-N-ethylacetamide that exhibits a single crystal X-ray crystallographic analysis at 150 K with crystal parameters that are approximately equal to the following:

16. Crystalline polymorph Form III of N-[3-(3-cyanopyrazolo[1,5a]pyrimidin-7yl) phenyl]-N-ethylacetamide.

17. The crystalline polymorph of claim 2, wherein the crystalline polymorph exhibits an X-ray powder diffraction pattern having characteristic peaks expressed in degrees 2θ at about 8.0 and 16.2 ± 0.2 °2θ.

18. The crystalline polymorph of claim 17, wherein the crystalline polymorph exhibits an X-ray powder diffraction pattern having characteristic peaks expressed in degrees 2θ at about 8. 0, 11.2, 16.2, 17.1, 17.6, 24.3, and 25.1 ± 0.2°2θ.

19. The crystalline polymorph of claim 18, wherein the crystalline polymorph exhibits an X-ray powder diffraction pattern substantially the same as that shown in Figure 11.

20. The crystalline polymorph of claim 2, wherein the crystalline polymorph exhibits chemical shifts in a ¹³C Solid State Nuclear Magnetic Resonance spectrum at about 12.1 and 12. 4 ± 0.2 ppm.

21. The crystalline polymorph of claim 2, wherein the crystalline polymorph exhibits chemical shifts in a C Solid State Nuclear Magnetic Resonance spectrum at about 22.8 and 25. 8 ± 0.2 ppm.

22. The crystalline polymorph of claim 2, wherein the crystalline polymorph exhibits a difference between the lowest ppm peak and another peak in a 13C Solid State Nuclear Magnetic Resonance spectrum of about 13.7 ppm.

23. The crystalline polymorph of claim 2, wherein the crystalline polymorph exhibits a chemical shift in a 13C Solid State Nuclear Magnetic Resonance at about 171.6 ± 0.2 ppm.

24. The crystalline polymorph of claim 23, wherein the crystalline polymorph exhibits chemical shifts in a ¹³C Solid State Nuclear Magnetic Resonance at about 12.1, 12.4, 22.8, 25.8, 44.1, 45.5, 79.0, 81.1, 111.0, 113.4, 131.4, 143.3, 145.7, 149.0, 150.1, 153.0, 155.5, and 171.6 ± 0.2 ppm.

25. The crystalline polymorph of claim 2, wherein the crystalline polymorph exhibits a difference between the lowest ppm peak and another peak in a ¹³C Solid State Nuclear Magnetic Resonance of about 159.5 ppm.

26. The crystalline polymorph of claim 25, wherein the crystalline polymorph exhibits delta values in a ¹³C Solid State Nuclear Magnetic Resonance spectrum of about 0.3, 10.7, 13.7, 32.0, 33.4, 66.9, 69.0, 98.9, 101.3, 119.3, 131.2, 133.6, 136.9, 138.0, 140.9, 143.4, and 159.5 ppm.

27. The crystalline polymorph of claim 2, wherein the crystalline polymorph exhibits an X-ray powder diffraction pattern substantially the same as that shown in Figure 11.

28. The crystalline polymorph of claim 2, wherein the crystalline polymorph exhibits a ¹³C Solid State Nuclear Magnetic Resonance spectrum substantial the same as that shown in Figure 12.

29. A pharmaceutical composition comprising a therapeutically effective amount of a hydrate crystalline polymorph of zaleplon and a pharmaceutically acceptable carrier or diluent.

30. The pharmaceutical composition of claim 29, wherein the pharmaceutical composition comprises at least about 90% by weight of Form II of zaleplon, based upon 100% total weight of zaleplon in the pharmaceutical composition.

31. The pharmaceutical composition of claim 30, wherein the pharmaceutical composition comprises at least about 95% by weight of Form II of zaleplon, based upon 100% total weight of zaleplon in the pharmaceutical composition.

32. The pharmaceutical composition of claim 29, wherein the pharmaceutical composition comprises at least about 90% by weight of Form III of zaleplon, based upon 100% total weight of zaleplon in the pharmaceutical composition.

33. The pharmaceutical composition of claim 32, wherein the pharmaceutical composition comprises at least about 95% by weight of Form III of zaleplon, based upon 100% total weight of zaleplon in the pharmaceutical composition.

34. A pharmaceutical composition comprising a therapeutically effective amount of the crystalline polymorph of claim 4 and a pharmaceutically acceptable carrier or diluent.

35. A pharmaceutical composition comprising a therapeutically effective amount of the crystalline polymorph of claim 5 and a pharmaceutically acceptable carrier or diluent.

36. A pharmaceutical composition comprising a therapeutically effective amount of the crystalline polymorph of claim 16 and a pharmaceutically acceptable carrier or diluent.

37. The pharmaceutical composition of claim 36, wherein the crystalline polymorph exhibits an X-ray powder diffraction pattern having characteristic peaks expressed in degrees 2 θ at about 8.0, 11.2, 16.2, 17.1, 17.6, 24.3, and 25.1 ± 0.2 °2θ.

38. A process for preparing Form II of zaleplon comprising:
(i) dissolving zaleplon in a non-aqueous solvent to form a solution; and
(ii) adding water to the solution.

39. A process for preparing Form III of zaleplon comprising:
(i) providing a solution containing zaleplon dissolved in an aqueous solvent; and
(ii) evaporating the solvent.

40. A method of treating anxiety in an animal in need thereof comprising administering an anti-anxiety effective amount of a crystalline polymorph of zaleplon, wherein the crystalline polymorph exhibits an X-ray powder diffraction pattern having characteristic peaks expressed in degrees 2 θ at about (a) 12.5 and (b) 21.2 or 21.4 ± 0.2 °2θ.

41. A method of treating epilepsy in an animal in need thereof comprising administering an anti-epilepsy effective amount of a crystalline polymorph of zaleplon, wherein the crystalline polymorph exhibits an X-ray powder diffraction pattern having characteristic peaks expressed in degrees 2 θ at about (a) 12.5 and (b) 21.2 or 21.4 ± 0.2 °2θ.

42. A method of inducing a sedative-hypnotic effect in an animal in need thereof comprising administering a sedative-hypnotic effective amount of a crystalline polymorph of zaleplon, wherein the crystalline polymorph exhibits an X-ray powder diffraction pattern having characteristic peaks expressed in degrees 2 θ at about (a) 12.5 and (b) 21.2 or 21.4 ± 0.2 °2θ.

43. A method of inducing muscle relaxation in an animal in need thereof comprising administering a skeletal muscle relaxing effective amount of a crystalline polymorph of zaleplon, wherein the crystalline polymorph exhibits an X-ray powder diffraction pattern having characteristic peaks expressed in degrees 2 θ at about (a) 12.5 and (b) 21.2 or 21.4 ± 0.2 °2θ.

44. The method of claim 40, wherein the crystalline polymorph exhibits an X-ray powder diffraction pattern having characteristic peaks expressed in degrees 2 θ at about 8.1, 11.0, 12.5, 13.3, 15.0, 16.8, 17.5, 18.0, 21.4, 22.2, 24.5, 25.1, 25.3, 25.7, 26.7, 27.1, 27.7, 28.2, and 30.3 ± 0.2 °2θ.

45. The method of claim 41, wherein the crystalline polymorph exhibits an X-ray powder diffraction pattern having characteristic peaks expressed in degrees 2 θ at about 8.1, 11.0, 12.5, 13.3, 15.0, 16.8, 17.5, 18.0, 21.4, 22.2, 24.5, 25.1, 25.3, 25.7, 26.7, 27.1, 27.7, 28.2, and 30.3 ± 0.2 °2θ.

46. The method of claim 42, wherein the crystalline polymorph exhibits an X-ray powder diffraction pattern having characteristic peaks expressed in degrees 2 θ at about 8.1, 11.0, 12.5, 13.3, 15.0, 16.8, 17.5, 18.0, 21.4, 22.2, 24.5, 25.1, 25.3, 25.7, 26.7, 27.1, 27.7, 28.2, and 30.3 ± 0.2 °2θ.

47. The method of claim 43, wherein the crystalline polymorph exhibits an X-ray powder diffraction pattern having characteristic peaks expressed in degrees 2 θ at about 8.1, 11.0, 12.5, 13.3, 15.0, 16.8, 17.5, 18.0, 21.4, 22.2, 24.5, 25.1, 25.3, 25.7, 26.7, 27.1, 27.7, 28.2, and 30.3 ± 0.2 °2θ.

48. The method of claim 40, wherein the crystalline polymorph exhibits an X-ray powder diffraction pattern having characteristic peaks expressed in degrees 2 θ at about 7.9, 10.6, 12.5, 14.8, 16.4, 16.8, 17.6, 21.2, 23.9, 24.1, 25.2, 25.5, 26.4, 27.0, 27.2, 27.4, and 28.3 ± 0.2 °2θ.

49. The method of claim 41, wherein the crystalline polymorph exhibits an X-ray powder diffraction pattern having characteristic peaks expressed in degrees 2 θ at about 7.9, 10.6, 12.5, 14.8, 16.4, 16.8, 17.6, 21.2, 23.9, 24.1, 25.2, 25.5, 26.4, 27.0, 27.2, 27.4, and 28.3 ± 0.2 °2θ.

50. The method of claim 42, wherein the crystalline polymorph exhibits an X-ray powder diffraction pattern having characteristic peaks expressed in degrees 2 θ at about 7.9, 10.6, 12.5, 14.8, 16.4, 16.8, 17.6, 21.2, 23.9, 24.1, 25.2, 25.5, 26.4, 27.0, 27.2, 27.4, and 28.3 ± 0.2 °2θ.

51. The method of claim 43, wherein the crystalline polymorph exhibits an X-ray powder diffraction pattern having characteristic peaks expressed in degrees 2 θ at about 7.9, 10.6, 12.5, 14.8, 16.4, 16.8, 17.6, 21.2, 23.9, 24.1, 25.2, 25.5, 26.4, 27.0, 27.2, 27.4, and 28.3 ± 0.2 °2θ.

52. A method of treating anxiety in an animal in need thereof comprising administering an anti-anxiety effective amount of the crystalline polymorph of claim 16.

53. A method of treating epilepsy in an animal in need thereof comprising administering an anti-epilepsy effective amount of the crystalline polymorph of claim 16.

54. A method of inducing a sedative-hypnotic effect in an animal in need thereof comprising administering a sedative-hypnotic effective amount of the crystalline polymorph of claim 16.

55. A method of inducing muscle relaxation in an animal in need thereof comprising administering a skeletal muscle relaxing effective amount of the crystalline polymorph of claim 16.

56. The method of claim 52, wherein the crystalline polymorph exhibits an X-ray powder diffraction pattern having characteristic peaks expressed in degrees 2 θ at about 8.0, 11.2, 16.2, 17.1, 17.6, 24.3, and 25.1 ± 0.2 °2θ.

57. The method of claim 53, wherein the crystalline polymorph exhibits an X-ray powder diffraction pattern having characteristic peaks expressed in degrees 2 θ at about 8.0, 11.2, 16.2, 17.1, 17.6, 24.3, and 25.1 ± 0.2 °2θ.

58. The method of claim 54 wherein the crystalline polymorph exhibits an X-ray powder diffraction pattern having characteristic peaks expressed in degrees 2 θ at about 8.0, 11.2, 16.2, 17.1, 17.6, 24.3, and 25.1 ± 0.2 °2θ.

59. The method of claim 55, wherein the crystalline polymorph exhibits an X-ray powder diffraction pattern having characteristic peaks expressed in degrees 2 θ at about 8.0, 11.2, 16.2, 17.1, 17.6, 24.3, and 25.1 ± 0.2 °2θ.
